(19) Europäisches Patentamt

European Patent Office

Office européen des brevets

(11) Veröffentlichungsnummer : **0 001 593**
**B1**

(12) # EUROPÄISCHE PATENTSCHRIFT

(45) Veröffentlichungstag der Patentschrift :
15.04.81

(21) Anmeldenummer : 78101068.1

(22) Anmeldetag : 05.10.78

(51) Int. Cl.³ : **C 07 C 67/05, C 07 C 69/16**

(54) **Verfahren zur Herstellung von Butendioldiacetat.**

(30) Priorität : **24.10.77 DE 2747634**

(43) Veröffentlichungstag der Anmeldung :
**02.05.79 (Patentblatt 79/09)**

(45) Bekanntmachung des Hinweises auf die Patenterteilung : **15.04.81 Patentblatt 81/15**

(84) Benannte Vertragsstaaten :
**BE CH DE FR GB NL**

(56) Entgegenhaltungen :
**DE - A - 2 200 124**
**DE - A - 2 611 423**
**US - A - 3 872 163**

**CHEMICAL ABSTRACTS,**
**Band 81, Nr. 3, 1974**
**Columbus, Ohio, U.S.A.**
**S. NAKAMURA et al. « Organic esters » Seite 263,**
**Abstract Nr. 12587g.**

(73) Patentinhaber : **BASF Aktiengesellschaft**
**Carl-Bosch-Strasse 38**
**D-6700 Ludwigshafen (DE)**

(72) Erfinder : **Weitz, Hans-Martin,Dr.**
**Auf dem Koeppel 40**
**D-6702 Bad Duerkheim 1 (DE)**
Erfinder : **Hartig, Juergen,Dr.**
**Londonder Ring 72**
**D-6700 Ludwigshafen (DE)**
Erfinder : **Schnabel, Rolf, Dr.**
**Tilsiter Strasse 9**
**D-6707 Schifferstadt (DE)**

## Verfahren zur Herstellung von Butendioldiacetat

Die Erfindung betrifft ein besonders wirtschaftliches Verfahren zur Herstellung von Butendiolacetat bzw. dessen Isomerengemisch durch umsetzung von Butadien, Essigsäure und Sauerstoff an einem Katalysator auf der Grundlage von Palladium oder Platin, bei dem anstelle von Butadien butadien-haltige Gemische verwendet werden, die bei der thermischen Zerlegung von höheren Kohlenwasserstoffe der Paraffinreihe oder bei der Dehydrierung von n-Butan- und/oder Buten-Gemischen anfallen. Das genannte Isomerengemisch enthält 2-Buten-1,4-dioldiacetat, 1-Buten-3,4-dioldiacetat und die entsprechenden Monoacetate bzw. Diole.

Die Umsetzung von reinem Butadien mit Sauerstoff und Essigsäure, an Platin- bzw. Palladium-Katalysatoren zu Buten-dioldiacetaten ist bekannt, z.B. aus den DE-OSen 22 17 452, 24 17 658 und 25 45 698. Für das im folgenden beschriebene Verfahren ist auf diese Schriften zu verweisen, soweit die Herstellung und Verwendung geeigneter Katalysatoren betriffen ist.

Einfach und zweifach ungesättigte Kohlenwasserstoffe können z.B. nach der DE-OS 22 00 124 und der US-PS 3 872 163 in Gegenwart palladium-haltiger Katalysatoren mittels Sauerstoff und freien Carbonsäuren in Gemische ungesättigter Carbonsäureester überführt werden. Man verwendet dabei Katalysatoren, die neben Palladium als Promotoren Salze des Lithium, Natriums oder Kaliums oder Verbindungen der Metalle Zink, Titan, Wismut, Cadmium oder Mangan enthalten. Die mit diesen Katalysatoren bei Gemischen von Butenen erzielbaren Ergebnisse befriedigen nicht in ihrer Selektivität, da Gemische ungesättigter Carbonsäureester erhalten werden, die u.a. einen unerwünscht hohen Anteil an Monoestern aufweisen.

Die DE-OS 25 09 251 beschreibt ebenfalls Umwandlungsreaktionen von Butenen in Gegenwart von Palladium, Sauerstoff und Essigsäure. Es war daher zu erwarten, daß Sauerstoff und Essigsäure sich mit Gemischen, die Butadien und z.B. 1-Buten enthalten, mindestens zu Mischungen von Butendiol-Diacetaten und Butenolmonoacetaten umsetzen würden. Dies gilt umso mehr, als in der DE-OS 26 27 001 beschrieben ist, daß die Umwandlung von Butadien in Butendioldiacetate von anderen ungesättigten Kohlenwasserstoffen, hier des Vinyl-cyclohexens sehr gestört wird, indem der Katalysator seine Aktivität verliert.

Es wurde nun gefunden, daß man bei der Herstellung von Butendioldiacetat bzw. dessen Isomerengemisch durch Umsetzung von Butadien, Sauerstoff und Essigsäure an einem Katalysator auf der Grundlage von Palladium oder Platin besonders vorteilhafte Ergebnisse erhält, wenn man anstelle von Butadien butadienhaltige Gemische verwendet, die bei der thermischen Zerlegung höherer Paraffinkohlenwasserstoffe oder bei der Dehydrierung von n-Butan- und/oder Buten-Gemischen anfallen und einen Katalysator einsetzt, der neben Palladium oder Platin untergeordnete Mengen an Tellur enthält.

Überraschenderweise werden hierbei aus den genannten Ausgangsgemischen praktisch ausschließlich Butendioldiacetat-Isomerengemische erhalten.

Butadien enthaltende Gemische, wie sie bei der thermischen Zerlegung (Pyrolyse) von höheren Kohlenwasserstoffen der Paraffinreihen entstehen, werden üblicherweise als « $C_4$-Schnitte » oder « $C_4$-Crackschnitte » bezeichnet.

Die nach der Erfindung zu verwendenden Katalysatoren enthalten Palladium oder Platin, vorzugsweise auf einem Aktivkohle enthaltenden Träger und daneben untergeordnete Mengen an Tellur.

Sofern der Anteil an acetylenisch ungesättigten Kohlenwasserstoffen (Propin, 1-Butin, Vinylacetylen) im Ausgangsgemisch nicht über 0,5 Volumenprozent liegt, verläuft die Reaktion nicht nur kurzzeitig, sondern praktisch ohne zeitlichen Aktivitätsverlust in der gewünschten Weise. Höhere Gehalten an Acetylen bringen die Reaktion über kurz oder lang zum Erliegen, während wechselnde Gehalte an Monoolefinen nicht stören.

Die Reaktion verläuft auch gut in Gegenwart wechselnder Wassermengen und in Gegenwart von Alkokolen ; man kann anstelle von Essigsäure ihre wäßrigen bzw. wäßrig-alkoholischen Lösungen verwenden. Nach den Angaben der DE-OS 26 11 423 kann man die Essigsäure in Form eines Essigsäureesters mit einem niedermolekularen Alkohol, beispielsweise Methanol, in die Reaktion einführen, wenn dort Bedingungen herrschen, bei denen eine Verseifungsreaktion möglich ist. Dieses Verfahren kann mit Vorteil bei der vorliegenden Erfindung angewandt werden.

Die Reaktionsgeschwindigkeit ist natürlich abhängig von der angebotenen Menge an Butadien. Sie ist jedoch bei gleichem Butadienangebot in weiten Grenzen unabhängig von der Menge der im $C_4$-Crackschnitt zusätzlich vorhandenen Kohlenwasserstoffe mit Ausnahme von acetylenisch ungesättigten Kohlenwasserstoffen.

Die bei der Reaktion anwesenden Begleiter des Butadiens werden nicht verändert und können anderen Zwecken zugeführt werden.

Die Reaktion kann in der Gasphase ebenso wie in Gas-Flüssiggemischen oder reiner Flüssigphase ablaufen.

Bevorzugt ist die Anwendung von Betriebstechniken, die reinen Flüssigbstrieb gestatten, d.h. mit gelösten $C_4$-Schnitten und gelöstem Sauerstoff arbeiten, weil sie als sicherer gelten.

Der Katalysator, der stets in fester Form anwesend ist, kann fest angeordnet oder suspendiert sein ; einschlägige Betriebstechniken sind bekannt.

Bei Ablauf in der Flüssigphase bzw. Gas-flüssig-Mischphase liegt die Reaktionstemperatur u.a. zwischen 70 und 125 °C, vorzugsweise 80 bis 110 °C, während die Gasphasenreaktion bei höherer Temperatur, z.B. 100 bis 180 °C, vorzugsweise 120 bis 150 °C abläuft.

Der Betriebsdruck wird weitgehend von der Verfahrenstechnik bestimmt und kann zwischen atmosphärischem Druck und z.B. 100 bar liegen ; für das Reaktionsgeschehen ist er nicht entscheidend. Der Gesamtdruck des Reaktionsgemisches in der Flüssigphase ergibt sich aus den Partialdrücken der Komponenten des Gemisches.

Typische $C_4$-Schnitte, wie sie z.B. bei den Verfahren auf treten, die in Ullmanns Enzyklopädie der technischen Chemie (4. Auflage Bd. *8* (1974), 158 und Bd. *9* (1975) 1 beschrieben sind, haben etwa folgende Zusammensetzung :

15 bis 80 % Butadien
20 bis 80 % Butene
1 bis 50 % Butane
0,01 bis 0,5 % acetylenisch ungesättigte Kohlenwasser stoffe (Propin, 1-Butin, Vinylacetylen) :

Falls ihr Gehalt an Acetylenen den Wert von etwa 0,5 % überschreitet, wäre für ein kontinuierliches Verfahren eine Reinigungsstufe vorzuschalten, die z.B. nach dem Verfahren der selektiven katalytischen Hydrierung verläuf.

Beispiel 1

12,5 g eines nach den Angaben der DE-OS 22 17 452, Beispiel 28 hergestellten Katalysators (5,7 % Pd, 1,0 % Te) werden in 600 ml Essigsäure in einem Rührkolben suspendiert. Bei 95 °C wird ein Gasgemisch aus 3 Nl/h $C_4$-Schnitt und 3 Nl/h Sauerstoff eingeleitet. Die Zusammensetzung des $C_4$-Schnitts ist aus der Tabelle, linke Spalte ersichtlich. Es ergibt sich eine Raum-Zeit-Ausbeute bei atmosphärischem Druck von 430 g Butendioldiacetaten pro kg Katalysator und Stunde ; vom eingeleiteten Butadien werden dabei 66 % in Diacetate umgewandelt. Die Isomerverteilung ist wie folgt :

68 % trans-2-Buten-1,4-diol-diacetat,
18 % cis-2-Buten-1,4-dioldiacetat,
14 % 1-Buten-3,4-dioldiacetat.

Tabelle

Zusammensetzung verwendeter $C_4$-Schnitte

| Kohlenwasserstoff | Beispiel 1 Vol.-% | Beispiel 2 Vol.-% |
|---|---|---|
| 1,3-Butadien | 34.8 | 45.7 |
| n-Butan | 6.1 | 4.8 |
| iso-Butan | 1.0 | 0.6 |
| 1-Buten | 17.9 | 13.6 |
| cis-2-Buten | 0.8 | 4.6 |
| trans-2-Buten | 2.6 | 7.5 |
| iso-Buten | 36.6 | 22.8 |
| Acetylene | 0.2 | 0.2 |
| übrige Kohlenwasserstoffe | Rest | Rest |

Beispiel 2

950 g des in Beispiel 1 verwendeten Katalysators werden, vermischt mit 3,5 l Glasringen (∅ 3 mm), in ein stählernes Reaktionsrohr mit 5 l Inhalt gefüllt. Pro Stunde werden 25 Nl Sauerstoff zusammen mit 0,8 l flüssigem, butadien-haltigem $C_4$-Schnitt (Zusammensetzung siehe Tabelle, rechte Spalte) gelöst in 5 l Essigsäure, bei 45 bar und 95 °C durch den Reaktor geleitet. Im gewonnenen Reaktionsgemisch sind 3,2 Gewichtsprozent trans-2-Buten-1,4-dioldiacetat, 0,6 Gewichtsprozent cis-2-Buten-1,4-dioldiacetat und 0,7 Gewichtsprozent 1-Buten-3,4-dioldiacetat enthalten ; dies entspricht einer Raum-Zeit-Ausbeute von 234 g Butendioldiacetaten pro kg Katalysator und Stunde.

In einem Vergleichsversuch, in dem anstelle von $C_4$-Schnitt 0,8 l/h reines flüssiges Butadien unter sonst gleichen Bedingungen verwendet werden, sind im Austrag 3,1 Gewichtsprozent des trans-Isomeren, 0,5 Gewichtsprozent des cis-Isomeren und 0,6 Gewichtsprozent des 3,4-Isomeren enthalten was einer Raum-Zeit-Ausbeute von 218 g/kg.h entspricht.

Beispiel 3

Man verfährt entsprechend den Angaben des Beispiels 2, jedoch werden pro Stunden 45 Nl Sauerstoff und 1,6 l flüssiger butadienhaltiger $C_4$-Schnitt (Zusammensetzung wie in Beispiel 2) gelöst in 5 l Essigsäure bei 65 bar und 95°C durch den Reaktor geleitet. Im gewonnenen Reaktionsgemisch sind 4,4 Gewichtsprozent trans-2-Buten-1,4-dioldiacetat, 0,6 Gewichtsprozent cis-2-Buten-1,4-dioldiacetat und 0,6 Gewichtsprozent 1-Buten-3,4-dioldiacetat enthalten ; dies entspricht einer Raum-Zeit-Ausbeute von 291 g/kg.h Butendioldiacetate.

In einem Vergleichsversuch, in dem anstelle von $C_4$-Schnitt 1,6 l/h reines flüssiges Butadien unter sonst gleichen Bedingungen eingesetzt werden, sind im ausreagierten Reaktionsgemisch 4,2 Gewichtsprozent des trans-Isomeren, 0,6 Gewichtsprozent des cis-Isomeren und 0,7 Gewichtsprozent des 3,4-Isomeren enthalten (Raum-Zeit-Ausbeute = 286 g/kg.h).

Beispiel 4

150 g des nach den Angaben der DE-OS 22 17 452 hergestellten Katalysators werden in ein Doppelmantelrohr (Durchmesser 35 mm, Länge 50 cm) gefüllt. Bei 130 °C werden pro Stunde 10 Nl butadienhaltiger $C_4$-Schnitt (Zusammensetzung wie in Beispiel 2), 10 Nl Sauerstoff und 250 ml Essigsäure zugeführt. Die Essigsäure wird in einem Verdampfer auf 130 °C erhitzt und dampfförmig eingeleitet. Es ergibt sich eine Raum-Zeit-Ausbeute von 97 g Butendioldiacetaten pro kg Katalysator und Stunde, wobei die Isomerenverteilung im wesentlichen der von Beispiel 1 gleicht.

In einem Vergleichsversuh, in dem anstelle von C$_4$-Schnitt 10 Nl/h reines Butadien unter sonst gleichen Bedingungen eingeleitet werden, liegt die Raum-Zeit-Ausbeute an Butendioldiacetaten bei 107 g/kg.h.

Beispiel 5

In einem 1 l-Rührautoklaven werden 400 g Methylacetat, 50 ml flüssiger C$_4$-Schnitt (Zusammensetzung entsprechend Beispiel 2), 96 g Wasser, 10 g eines auf der Grundlage eines sulfonierten, vernetzten Polystyrols aufgebauten, stark sauren Ionenaustauschers (Lewatit S 100) und 6 g des in Beispiel 1 verwendeten Palladium und Tellur enthaltenden Katalysators unter einem Sauerstoffdruck von 20 bar 4 Stunden auf 85 °C erhitzt. Das gewonnene Reaktionsgemisch enthält 10,6 g Butendiole und deren Ester, entsprechend einer Raum-Zeit-Ausbeute von 440 g Butendioldiacetaten pro kg Katalysator und Stunde.

In einem Vergleichsversuch, in dem anstelle von C$_4$ Crackschnitt 50 ml reines flüssiges Butadien unter sonst gleichen Bedingungen eingesetzt werden, sind im gewonnenen Reaktionsgemisch 13,0 g Butendiolverbindungen (berechnet als Diacetate) enthalten, entsprechend einer Raum-Zeit-Ausbeute von 540 g/kg.h.

Beispiel 6

556 g des in Beispiel 1 verwendeten Katalysators werden —abgefüllt in 60 zylindrischen Behältern, deren Stirnflächen mit Sieben abgedeckt sind— zusammen mit 1 170 g eines stark sauren Ionenaustauschers auf der Grundlage sulfonierten, vernetzten Polystyrols —ebenfalls abgefüllt in 60 g gleichartigen Behältern— in ein Reaktionsrohr mit 5 l Inhalt gegeben ; dabei werden jeweils 8 Behälter mit Ionenaustauscher überschichtet mit 8 Katalysatorgefüllten Behältern.

Bei 90 bar und 105 °C werden pro Stunde 25 Nl Sauerstoff, 0,8 l flüssiger C$_4$-Schnitt (Zusammensetzung entsprechend Beispiel 2) und 0,1 l Wasser, gelöst in 10,3 l Methylacetat durch den Reaktor geleitet. Im ausreagierten Reaktionsgemisch sind 1,0 Gewichtsprozent trans-2-Buten-1,4-dioldiacetat, 0,2 Gewichtsprozent cis-2-Buten-1,4-dioldiacetat und 0,3 Gewichtsprozent 1-Buten-3,4-dioldiacetat enthalten ; das entspricht einer Raum-Zeit-Ausbeute von 254 g Butendioldiacetaten pro kg Katalysator und Stunde. Der Gesamtgehalt an Monoacetaten und Diolen liegt unter 0,1 Gewichtsprozent.

In einem Vergleichsversuch, in dem anstelle von C$_4$-Schnitt 0,8 l/h reines flüssiges Butadien unter sonst gleichen Bedingungen eingesetzt werden, sind im Reaktionsgemisch 1,1 Gewichtsprozent des trans-Isomeren, 0,2 Gewichtsprozent des cis-Isomeren und 0,3 Gewichtsprozent des 3,4-Isomeren enthalten (Raum-Zeit-Ausbeute 263 g/kg.h ; Gehalt an Monoacetaten und Diolen <0,1 Gewichtsprozent).

Beispiel 7

100 g Methylacetat, 4,3 ml Wasser, 10 g des in Beispiel 1 erwähnten Katalysators und 10 g eines in der H$^+$-Form vorliegenden Ionenaustauschers vom Mordenit-Typ (Zeolon H 100) als Verseifungskatalysator werden in einen Stahlautoklaven gefüllt. Nachdem 10 g Butadien-haltiger C$_4$-Schnitt zugegeben sind, werden 100 bar Sauerstoff aufgepreßt und im Laufe von 2 Stunden unter Schütteln bis auf 85 °C aufgeheizt. im Filtrat, das nach Abkühlen und Entspannen des Autoklaven gewonnen wird, sind 1,6 g trans-2-Buten-1,4-dioldiacetat, 0,2 g cis-2-Buten-1,4-dioldiacetat und 0,4 g 1-Buten-3,4-dioldiacetat enthalten, während Monoacetate und Diole insgesamt in weniger als 0,1 g vorliegen.

Im Filtrat des Vergleichsversuchs, in dem anstelle von C$_4$-Schnitt 10 g reines Butadien unter sonst gleichen Bedingungen eingesetzt werden, liegen 1,2 g des trans-Isomeren, 0,1 g des cis-Isomeren und 0,3 g des 3,4-Isomeren vor (Monoacetate und Diole weniger als 0,1 g).

**Ansprüche**

1. Verfahren zur Herstellung von Butendioldiacetat bzw. dessen Isomerengemisch durch Umsetzung von Butadien, Sauerstoff und Essigsäure an einem Katalysator auf der Grundlage von Palladium oder Platin, *dadurch gekennzeichnet,* daß man anstelle von Butadien butadienhaltige Gemische verwendet, die bei der thermischen Zerlegung höherer Paraffinkohlenwasserstoffe oder bei der Dehydrierung von n-Butanund/oder Buten-Gemischen anfallen, und einen Katalysator einsetzt, der neben Palladium oder Platin untergeordnete Mengen an Tellur enthält.

2. Verfahren nach Anspruch 1, *dadurch gekennzeichnet,* daß man butadienhaltige Gemische von C$_4$-Kohlenwasserstoffen verwendet, die nicht mehr als 0,5 Volumenprozent acetylenisch ungesättigte Kohlenwasserstoffe (bezogen auf das gasförmige Gemisch) enthalten.

**Claims**

1. A process for the manufacture of butenediol diacetate or a butenediol diacetate isomer mixture by reacting butadiene, oxygen and acetic acid over a catalyst based on palladium or platinum, *characterized in that* instead of butadiene there is used a butadiene-containing mixture which is formed in the thermal cracking of higher paraffin hydrocarbons or in the dehydrogenation of n-butane and/or butene mixtures, and a catalyst is used which contains minor amounts of tellurium in addition to palladium or platinum.

2. A process as claimed in claim 1, *characterized in that* a butadiene-containing mixture of $C_4$-hydrocarbons, which contains not more than 0.5 per cent by volume (based on the gaseous mixture) of acetylenically unsaturated hydrocarbons, is used.

### Revendications

1. Procédé pour la préparation de diacétate de butènediol ou de mélange de ses isomères par réaction de butadiène, d'oxygène et d'acide acétique sur un catalyseur à base de palladium ou de platine, caractérisé en ce qu'on utilise, à la place de butadiène, des mélanges contenant du butadiène, puis se forment lors de la décomposition thermique d'hydrocarbures paraffiniques supérieurs ou lors de la déshydrogénation de mélanges de n-butane et/ou de butène, et en ce qu'on utilise un catalyseur qui contient, outre le palladium ou le platine, des quantités mineures de tellure.

2. Procédé selon la revendication 1, caractérisé en ce qu'on utilise des mélanges d'hydrocarbures en $C_4$ contenant du butadiène, qui ne contiennent pas plus de 0,5 % en volume d'hydrocarbures insaturés acétyléniquement (par rapport au mélange gazeux.